# EUROPEAN PATENT SPECIFICATION

(11) **EP 0 552 526 B1**
(45) Date of publication and mention of the grant of the patent: **29.10.1997**
(21) Application number: 92300568.0
(22) Date of filing: 23.01.1992
(51) Int. Cl.: A61B 10/00, A61B 17/34

(54) **Surgical device for volumetric localization, biopsy and surgical procedures**
Chirurgisches Instrument für Volumenortung, Biopsie und chirurgische Verfahren
Dispositif chirurgical pour la localisation volumétrique, la biopsie et les procédés chirurgicaux

(43) Date of publication of application: 28.07.1993
(73) Proprietor: Mosby, Richard A, Houston, Texas 77025 (US)
(72) Inventor: Mosby, Richard A, Houston, Texas 77025 (US)
(74) Representative: Jones, William

(56) References cited:
- EP-A- 0 160 870
- DE-C- 25 796
- GB-A- 2 066 668
- GB-A- 2 116 046
- US-A- 2 198 319
- US-A- 4 927 426
- US-A- 4 966 583

## Description

### BACKGROUND OF THE INVENTION

### FIELD OF THE INVENTION

This invention relates to surgical devices and more particularly to a surgical device for volumetric localization, biopsy, and surgical procedures.

### DESCRIPTION OF THE PRIOR ART

Presently available surgical devices for localization, biopsy, and surgical procedures use the introduction of a needle directly through the skin and are usually linear in operation. This results in haphazard localization, biopsy and surgical procedures. Tissue stabilization, tissue return and tissue amounts are not consistent. These devices offer no consideration of a third dimension in evaluation, i.e., the amount, volume or extent of a lesion, tumor or other tissue mass.

For example, in mammography, presently available localization needles are linear and placed at or near the lesion to mark the site of surgical removal. These may be stabilized with a securing barb within the breast or externally by tape or securing suture. The tip of the marking needle may move making the site incorrect for biopsy. The surgeon then follows down the path of the needle in the breast to the site of the marking tip of the localization needle. There the surgeon removes an amount of tissue based on a visual impression of the extent of the mass. This is sent to the pathologist or radiologist for evaluation as to the completeness of removal of the mass. If removal is insufficient, the surgeon has to return and remove more tissue using the same imprecise visual impression as to size and position of the tissue mass.

In biopsy procedures, presently available biopsy needles are linear and tubular and result in limited tubular biopsy material when placed into lesions, tumours and other tissue masses. This results in variable small amounts of tissue being made available for evaluation. Occasionally, no tissue is returned requiring repeat attempts causing unnecessary repeat trauma on reinsertion.

In surgery, presently available indirect surgical devices, as in suction devices, are placed into tissues and indiscriminate tissue removal takes place. Portions of organs not requiring removal are removed and/or damaged in this method. Consequently, there has been a need to provide a surgical device that allows consideration or volume, amount and/or extent of a lesion.

The most relevant forms of prior art will now be described. DEBBAS US Patent 4 966 583 discloses a mass localisation device which comprises inter alia a catheter, needle and balloon. In use the combination of the needle and catheter are inserted into body issue and under assistance of x-ray radiography, the device is moved towards a mass in the issue, subsequently, the balloon is inflated for contacting the mass which makes the mass palpable.

Van-Tech Incorporated in EP-A-160 870 disclose a medical retrieval device sized and adapted for use through the working channel of an endoscope for removing stones and the like from kidneys, ureter and/or biliary ducts.

Porges GB-A-2 066 668 discloses a surgical extractor for removing foreign bodies from natural passages and comprises a cage or basket formed by steel wires which are retractable in side a tube.

In the specific description of this specification, the passages relating to surgical procedures are indicated as exemplary only.

### Summary of the Invention

One of the objects of the present invention to provide a new and improved surgical device for the localization/removal of tissue in the human body for evaluation, ie, for localization, biopsy and surgical procedures.

Another object of the invention is to provide a new and improved surgical device for the localization/removal of tissue in the human body for evaluation, i.e., for localization, biopsy and surgical procedures, having means to outline or encase the entire volume or amount of tissue required to be removed.

Another object of the invention is to provide a new and improved surgical device for the localization/removal of tissue in the human body for evaluation, i.e., for localization, biopsy and surgical procedures, having a tubular shaft enclosing a needle array which is introduced into the body adjacent to a tumor or tissue mass and when the site of a mass is reached, the needle array is deployed into a configuration that outlines or encases the entire volume or amount of tissue required to be removed.

Still another object of the invention is to provide a new and improved surgical device for the localization/removal of tissue in the human body for evaluation, i.e., for localization, biopsy and surgical procedures, that employs a tubular shaft enclosing a preloaded needle array which is introduced into the body adjacent to a tumor or tissue mass and when the site of a mass is reached, the pre-loaded needle array is deployed into a predetermined configuration that outlines or encases the entire volume or amount of tissue required to be removed.

Still another object of the invention is to provide a new and improved surgical device for the localization/removal of tissue in the human body for evaluation, i.e., for localization, biopsy and surgical procedures, that employs a tubular shaft enclosing a preloaded needle array which is introduced into the body adjacent to a tumor or tissue mass and when the site of a mass is reached, the pre-loaded needle array is deployed into a predetermined configuration to outline, encase and limit the available tissue for removal to insure only the required volume or amount of tissue is removed.

Still another, object of the invention is to provide a new and improved surgical device for the localization/removal of tissue in the human body for evaluation, i.e., for localization, biopsy and surgical procedures, that employs a tubular shaft enclosing a preloaded needle array which is introduced into the body adjacent to a tumor or tissue mass and when the site of a mass is reached, the pre-loaded needle array is deployed into a predetermined configuration to outline, encase and limit and exclude tissue in the human body for surgical removal by conventional means such as electrocautery or surgical excision or by newer means such as chemical, laser and sonic surgical means.

Still another object of the invention is to provide a new and improved surgical device for the localization/removal of tissue in the human body for evaluation, i.e. for localization biopsy and surgical procedures, that employs a tubular shaft enclosing a preloaded needle array which is introduced into the body adjacent to a tumour or tissue mass and when the site of a mass is reached, the pre-loaded needle array is deployed into a predetermined configuration to outline, encase and limit and exclude tissue in the human body for surgical removal by standard direct surgical means or through a small opening.

Another object of this invention is to decrease the deformity caused by surgical intervention by decreasing the amount of tissue removed and the size of the surgical entry site.

Other objects of the invention will become apparent from time to time throughout the specification and claims as hereinafter related.

The above noted objects and other objects of the invention are accomplished according to the invention by a surgical device for volumetric localisation, biopsy and surgical procedures comprising a straight hollow tubular shaft (1) of a size easily inserted into the human body through or adjacent to a tumour or tissue mass, a filament array (3) positioned in said straight hollow shaft (1) comprising a plurality of filaments of a spring deformable material having a substantially curved shape when unstressed and when straightened and positioned in said shaft being preloaded by the spring properties of the filaments, and means (2) supporting said array of filaments and operable to move the same relative to said hollow shaft to extend said filaments from said hollow shaft to an unstressed position where said filaments assume said substantially curved shape for outlining, encasing and excluding a known volume of tumour or other tissue mass from the human body for the purpose of localisation, biopsy or surgical removal, characterised in that the filament array (3) comprises a plurality of individual filaments each filament being attached at one end to the supporting means (2), the filaments being adapted to curl into a substantially closed loop in their unstressed condition and are straightened and inserted into tubular shaft (1) for use in a preformed and preloaded condition.

Preferably, the supporting means (2) comprises a stylet (2) movable longitudinally in said hollow shaft (1) supporting said filament array (3) for movement into and out of said hollow shaft (1). This arrangement ensures that the insertion area is kept to a minimum and therefore, any potential scarring is also kept to a minimum.

Preferably, the hollow shaft (1), filament array (3) and stylet (2) are formed of surgical steel or plastic. This has the advantage that each element of the device can be constructed of materials that are likely to provide them with the longest life expectancy. This is especially important in the light of the fact that parts of the device may be subjected to, for example a lasering effect.

Preferably, some of the filaments that form the filament array (3) are cutting filaments (8). This has the advantage that human tissue encased by the cutting filaments can be macerated without having to resort to conventional surgical techniques that may lead to excessive scarring.

Preferably, the device is provided with a filament tip control (7) that captures the tips of the cutting filaments (8) and secures them. This ensures that maceration of tissue can be controlled typically through a single pulling action of the control stylet.

Preferably, the device further comprises a laser device (11) provided with a laser control stylet (10) to guide a laser beam into the area encased by the filament array (3). This has the advantage that only tissue encased by the filament array is subjected to the laser treatment.

Preferably, the device further comprises a sound transducer (14) and a sound control stylet (13), the sound transducer (14) being capable of emitting sound waves (15) introduced through the stylet (2) by the sound control stylet (13), into the mass of exposed tissue (9) limited by the filament array (3), the sound waves acting within a pre-determined encircling configuration of the filament array (3) to annihilate the volume of tissue (9) encased by the filament array (3). This has the advantage that only tissue encased by the filament array is subjected to the sound wave treatment.

### BRIEF DESCRIPTION OF THE DRAWINGS

FIG. 1 is a side view of a surgical device for volumetric localization, biopsy and surgical procedures illustrating a preferred embodiment of the invention having preformed and preloaded filaments inside a tubular shaft prior to being deployed in use.

FIG. 2 is a side view of the surgical device of FIG. 1 in its deployed state with the filament array released into their functional position for encasing a tumor or other tissue mass.

FIG. 3 is a side view of the surgical device of FIG. 1 in use with the tubular shaft entering the breast and passing through a tissue mass with the control stylet and the filament array poised prior to release.

FIG. 4 is a side view of the surgical device of FIG. 1 in use with the tubular shaft withdrawn allowing release of the filament array into a functional position concentrically outlining and encasing a tissue mass in the breast.

FIG. 5 is a side view of the surgical device of FIG. 1 in use with the tubular shaft withdrawn allowing release of the filament array into a functional position eccentrically outlining encasing a tissue mass in the breast.

FIG. 6 is a side view of the surgical device of FIG. 1 in use with the tubular shaft totally removed and the filament array deployed to isolate the tissue mass from the breast tissue allowing a surgeon to remove the deployed filament array by following the control stylet into the area of the tissue mass.

FIG. 7 is a view of an expanded use of the surgical device of FIG. 1 wherein the filament array outlines and encases a volume of tissue after being deployed and the filaments function as cutting filaments.

FIG. 8 shows the surgical device of FIG. 7 with a filament tip control capturing the tips of the cutting filaments and securing them so that pulling the cutting control stylet macerates the tissue mass the arc they subtend is decreased.

FIG. 9 shows a further expansion of the surgical device of FIG. 1 with a laser device emitting laser light introduced through the control stylet into the mass of exposed tissue and limited by the encasing filament array.

FIG. 10 shows a further expansion of the surgical device of FIG. 1 with a sound transducer emitting sound waves introduced through the control stylet into the mass of exposed tissue and limited by the encasing filament array.

### DESCRIPTION OF THE PREFERRED EMBODIMENT

In the various views, a surgical device is shown for localizing, biopsy or surgical excision of masses in the human body. The device is used to outline, encase and exclude tissue from the human body for the purpose of removal from the human body at the time of use or at a later time. The volumetric localization/biopsy/surgical device consists of a tubular shaft to enter the human body. Within the tubular shaft is a filament array that is pre-loaded to deploy into a predetermined configuration to provide an area outlining, encasing or excluding human tissue. After deployment of the filament array into an excluding arrangement, conventional and non-conventional forms of surgical excision may be used to remove tissue excluded by the filament array simply by introducing the source of this form of surgical excision through the control stylet and exposing the encased tissue to the surgical source.

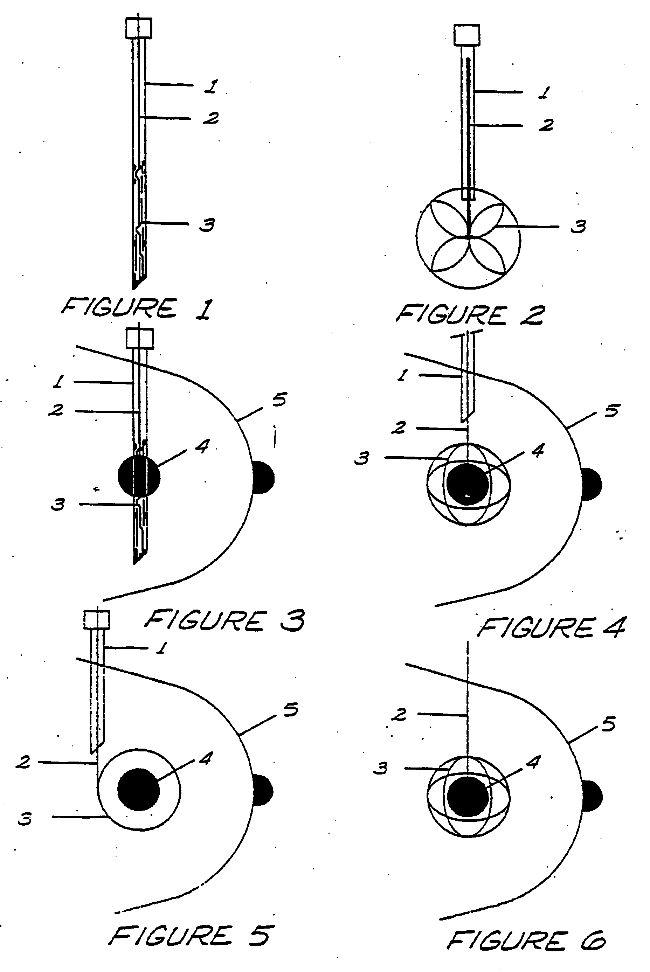

In FIG. 1 there is shown a side view of a surgical device for volumetric localization, biopsy and surgical procedures. The surgical device comprises a tubular shaft or needle 1 for insertion into human tissue. Tubular shaft 1 encloses a control stylet 2 which supports, manipulates and controls a filament array 3 which is shown in its charged or "ready" state. Filament array 3 comprises a plurality of individual filaments which curl into a substantially closed loop in their unstressed condition and are straightened and inserted into tubular shaft 1 for use in a preformed and preloaded condition.

In FIG. 2 the surgical device is shown in its deployed state with the tubular shaft 1, the control stylet 2, and the filament array 3 shown with the preformed and pre-loaded filaments released into their functional position. In this position, control stylet 2 has been moved to push the filament array 3 outside the end of tubular shaft 1 (or the shaft 1 has been withdrawn from stylet 2) where the individual filaments curl into substantially closed loops for outlining, encasing and excluding tissue from the human body.

In FIG. 3, the surgical device is shown in use with the tubular shaft 1 entering the breast 5 and passing through a tissue mass 4 with the control stylet 2 and the filament array 3 poised prior to release. In FIG. 4, the surgical device is shown with the tubular shaft 1 withdrawn allowing release of the filament array 3 into its functional position by advancing the control stylet 2 to concentrically outline and encase a mass 4 in the breast 5.

The surgical device described above is constructed of metal, plastic and/or other durable materials having properties which permit optimum advantage to the various units of the composite. For example, tubular shaft 1 can be made of metal to control the spring energy of the preloaded filament array 3 which may be made of spring steel or spring plastic. The individual filaments may be coated with materials to enhance their limiting properties for use as with laser beam annihilation (laser surgery) as described below. The control stylet 2 may be made of steel or plastic, etc.

The surgical device described above, in a best use conventional surgical situation, allows a surgeon to outline the extent of a lesion, tumor or other tissue mass, prior to surgery and remove only involved tissue or tissue of concern by simply removing the device, leaving uninvolved and/or adjacent tissue undisturbed. In a best use nonconventional surgical application, laser, chemical, sound, etc., may be used as agents. The localization/biopsy/surgical device limits tissue exposure to these modalities to that within the devices. The device may then be removed through the tubular shaft requiring only a small external opening.

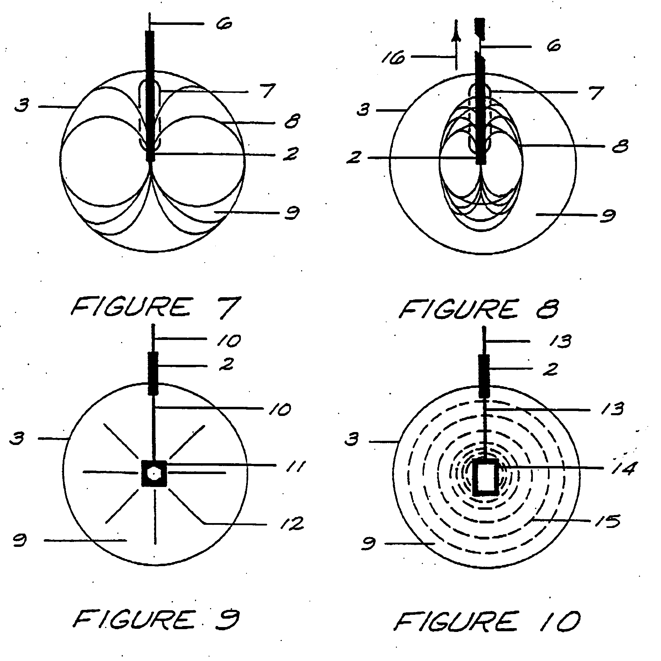

In FIG. 3, the surgical device is shown in use with the tubular shaft 1 entering the breast 5 and passing through a tissue mass 4 with the control stylet 2 and the filament array 3 poised, prior to release. In FIGS. 4 and 6, the surgical device is shown with the tubular shaft 1 withdrawn allowing release of the filament array 3 into its functional position by advancing the control stylet 2 to concentrically outline and encase a mass 4 in the breast 5.

In FIG. 5, the surgical device is placed eccentrically to the tissue mass 4 in the breast 5. In this view, the tubular shaft 1 is withdrawn allowing release of the filament array 3 into its functional, i.e., closed loop, position by advancing control stylet 2 and eccentrically outlining and encasing a tissue mass 4 in the breast 5. In FIG. 6, the surgical device is shown in use with the tubular shaft 1 totally removed from filament array 3. This localizes a mass 4 in the breast 5 with the filament array 3 deployed to surround and isolate the mass 4 from the breast tissue 5. The surgeon may then remove the deployed filament array 3 and encased tissue mass 4 by following the control stylet 2 into the area of the mass 4.

In FIGS. 7 and 8, the surgical device is shown with the filaments used to perform a surgical function. In FIG. 7, the filament array 3 outlines and encases a volume of tissue 9 after being deployed with the control stylet 2 as described above. The apparatus has cutting filaments 8 deployed by the control stylet 6. In FIG. 8, the filament tip control 7 captures the tips of the cutting filaments 8 and secures them. Pulling the cutting control stylet 6 in the direction 16 macerates the human tissue 9 by cutting as the arc they subtend is decreased. The tissue 9 exposed to this cutting is limited by the area enclosed by the filament array 3 deployed by, the control stylet 2.

FIG. 9 shows a further expansion of the surgical device with a laser device 11 fitting laser light 12 introduced through the control stylet 6 by the laser control stylet 10 into the mass of exposed tissue 9 limited by the filament array 3. In the embodiment of FIG. 9, the laser beam is restricted by the predetermined, i.e., encircling, configuration of the filament array 3 while allowing annihilation of the volume of tissue encased by the filament array 9.

FIG. 10 shows a further expansion of the surgical device with a sound transducer 14 emitting sound waves 15 introduced through the control stylet 2 by the sound control stylet 13 into the mass of exposed tissue 9 limited by the filament array 3. In the embodiment of FIG. 10, the sound waves are is restricted by the predetermined, i.e., encircling, configuration of the filament array 3 while allowing annihilation of the volume of tissue encased by the filament array 9.

While this invention has been shown fully and completely with special emphasis on certain preferred embodiments, it should be understood that within the scope of the claims the invention may be practiced otherwise than as specifically described herein.

## Claims

1. A surgical device for volumetric localisation, biopsy and surgical procedures comprising a straight hollow tubular shaft (1) of a size easily inserted into the human body through or adjacent to a tumour or tissue mass, a filament array (3) positioned in said straight hollow shaft (1) comprising a plurality of filaments of a spring deformable material having a substantially curved shape when unstressed and when straightened and positioned in said shaft being preloaded by the spring properties of the filaments, and means (2) supporting said array of filaments and operable to move the same relative to said hollow shaft to extend said filaments from said hollow shaft to an unstressed position where said filaments assume said substantially curved shape for outlining, encasing and excluding a known volume of tumour or other tissue mass from the human body for the purpose of localisation, biopsy or surgical removal, characterised in that the filament array (3) comprises a plurality of individual filaments each filament being attached at one end to the supporting means (2), the filaments being adapted to curl into a substantially closed loop in their unstressed condition and are straightened and inserted into tubular shaft (1) for use in a preformed and preloaded condition.

2. A surgical device according to claim 1 characterised in that the supporting means (2) comprises a stylet (2) movable longitudinally in said hollow shaft (1) supporting said filament array (3) for movement into and out of said hollow shaft (1).

3. A surgical device according to claims 1 and 2 characterised in that the hollow shaft (1), filament array (3) and stylet (2) are formed of surgical steel or plastic.

4. A surgical device according to any of the preceding claims characterised in that some of the filaments that form the filament array (3) are cutting filaments (8).

5. A surgical device as claimed in claim 4 characterised in that the device is provided with a filament tip control (7) that captures the tips of the cutting filaments (8) and secures them.

6. A surgical device as claimed in claims 1-3 characterised in that the device further comprises a laser device (11) provided with a laser control stylet (10) to guide a laser beam into the area encased by the filament array (3).

7. A surgical device as claimed in claims 1-3 characterised in that the device further comprises a sound transducer (14) and a sound control stylet (13), the sound transducer (14) being capable of emitting sound waves (15) introduced through the stylet (2) by the sound control stylet (13), into the mass of exposed tissue (9) limited by the filament array (3), the sound waves acting within a pre-determined encircling configuration of the filament array (3) to annihilate the volume of tissue (9) encased by the filament array (3).

## Patentansprüche

1. Eine chirurgische Vorrichtung für volumetrische Lokalisations-, Biopsie- und Operationsverfahren, die folgendes umfaßt: einen geraden, hohlen, röhrenförmigen Schaft (1) mit geeigneten Abmessungen zur leichten Einführung in den menschlichen Körper durch eine oder an einer Tumor- oder Gewebemasse, eine in besagtem geraden, hohlen Schaft (1) positionierte Filamentanordnung (3), die eine Mehrzahl von Filamenten aus einem federnd deformierbaren Material, das in entspanntem Zustand eine wesentlich bogenförmige Gestalt hat und nach Ausrichtung und Positionierung in besagtem Schaft durch die federnden Eigenschaften der Filamente vorgespannt wird, und Mittel (2) zur Abstützung besagter Filamentanordnung aufweist, die dazu fähig sind, dieselbe im Verhältnis zu besagtem hohlen Schaft zu bewegen, um besagte Filamente von besagtem hohlen Schaft in einen entspannten Zustand zu strecken, in welchem besagte Filamente die besagte, wesentlich bogenförmige Gestalt annehmen, um ein bekanntes Volumen eines Tumors oder einer anderen Gewebemasse im Umriß zu bestimmen, zu umgeben und aus dem menschlichen Körper zwecks Lokalisation, Biopsie oder operativer Entfernung zu exkludieren, dadurch gekennzeichnet, daß die Filamentanordnung (3) eine Mehrzahl individueller Filamente aufweist, wobei jedes Filament an einem Ende an dem Stützmittel (2) angebracht ist und die Filamente adaptiert sind, um sich in ihrem entspannten Zustand in eine wesentlich geschlossene Schleife zu krümmen und bei Einfügung in einen röhrenförmigen Schaft (1) geradegerichtet werden, um in einem vorgeformten und vorgespannten Zustand verwendet zu werden.

2. Eine chirurgische Vorrichtung entsprechend Anspruch 1, dadurch gekennzeichnet, daß das Stützmittel (2) einen Mandrin (2) umfaßt, der in besagtem hohlen Schaft (1), der besagte Filamentanordnung (3) unterstützt, längs bewegt werden kann, um in besagten hohlen Schaft (1) hinein- und aus diesem herausbewegt zu werden.

3. Eine chirurgische Vorrichtung entsprechend Ansprüchen 1 und 2, dadurch gekennzeichnet, daß der hohle Schaft (1), die Filamentanordnung (3) und der Mandrin (2) aus für operative Zwecke geeignetem Stahl oder Kunststoff geformt sind.

4. Eine chirurgische Vorrichtung entsprechend einem der vorstehenden Ansprüche, dadurch gekennzeichnet, daß manche der Filamente, die die Filamentanordnung (3) bilden, Schneidfilamente (8) sind.

5. Eine chirurgische Vorrichtung entsprechend Anspruch 4, dadurch gekennzeichnet, daß die Vorrichtung mit einer Filamentspitzenregelung (7) versehen ist, die die Spitzen der Schneidfilamente (8) einfängt und sichert.

6. Eine chirurgische Vorrichtung entsprechend Ansprüchen 1 - 3, dadurch gekennzeichnet, daß die Vorrichtung ferner eine Laservorrichtung (11) mit einem Lasersteuermandrin (10) aufweist, um einen Laserstrahl in den Bereich zu leiten, der von der Filamentanordnung (3) umgeben ist.

7. Eine chirurgische Vorrichtung entsprechend Ansprüchen 1 - 3, dadurch gekennzeichnet, daß die Vorrichtung ferner einen Schallkopf (14) und einen Tonregelmandrin (13) umfaßt, wobei der Schallkopf (14) über den Mandrin (2) eingeführte Schallwellen (15) durch den Tonregelmandrin (13) in die Masse freigelegten, von der Filamentanordnung (3) umgebenen Gewebes (9) aussenden kann und wobei die Schallwellen ihre Wirkung innerhalb einer vorbestimmten, einfassenden Konfiguration der Filamentanordnung (3) entfalten, um das von der Filamentanordnung (3) umgebene Gewebevolumen (9) zu vernichten.

## Revendications

1. Dispositif chirurgical pour la localisation volumétrique, les biopsies et les procédures de chirurgie comprenant une tige droite tubulaire creuse (1) d'une dimension pouvant être facilement insérée dans le corps humain à travers ou à côté d'une tumeur ou d'une masse de tissus, d'un ensemble de filaments (3) positionné dans ladite tige droite creuse (1) comprenant une pluralité de filaments en matériau élastique déformable ayant une forme essentiellement courbe à l'état libre et, lorsque redressé et positionné dans ladite tige, est préchargé par les propriétés élastiques des filaments, et un moyen (2) de support du dit ensemble de filaments pouvant être opéré pour déplacer celui-ci par rapport à ladite tige creuse pour faire sortir lesdits filaments de ladite tige creuse et prendre une position à l'état libre dans laquelle lesdits filaments reprendront leur dite forme essentiellement courbe pour suivre le contour, enfermer et exclure un volume connu de tumeur ou de masse de tissus du corps humain à des fins de localisation, de biopsie ou d'ablation chirurgicale, caractérisé en ce que l'ensemble de filaments (3) comprend une pluralité de filaments individuels chaque filament étant attaché à une extrémité au moyen de support (2), les filaments étant adaptés pour s'enrouler en une boucle essentiellement fermée à l'état libre et sont redressés et insérés dans la tige tubulaire (1) pour utilisation dans un état préformé et préchargé.

2. Dispositif chirurgical selon la revendication 1 caractérisé en ce que le moyen de support (2) comprend un stylet (2) déplaçable longitudinalement dans ladite tige creuse (1) supportant ledit ensemble de filaments (3) pour les faire rentrer ou sortir de ladite tige creuse (2).

3. Dispositif chirurgical selon les revendications 1 et 2 caractérisé en ce que la tige creuse (1), l'ensemble de filaments (3) et le stylet (2) sont formés en acier ou plastique chirurgical.

4. Dispositif chirurgical selon l'une quelconque des revendications précédentes caractérisé en ce que certains des filaments formant l'ensemble de filaments (3) sont des filaments coupants (8).

5. Dispositif chirurgical selon la revendication 4 caractérisé en ce que le dispositif est doté d'un contrôle de pointe de filament (7) qui capture et fixe les pointes des filaments coupants (8).

6. Dispositif chirurgical selon les revendications 1 à 3 caractérisé en ce que le dispositif comprend de plus un dispositif laser (11) doté d'un stylet de contrôle laser (10) pour guider le faisceau laser dans la zone enfermée par l'ensemble de filaments (3).

7. Dispositif chirurgical selon les revendications 1 à 3 caractérisé en ce que le dispositif comprend de plus un transducteur sonore (14) et un stylet de contrôle de son (13), le transducteur sonore (14) étant capable d'émettre des ondes sonores (15) introduites à travers le stylet (2) par le stylet de contrôle de son (13) dans la masse de tissus exposés (9) délimitée par l'ensemble de filaments (3), les ondes sonores agissant dans une configuration entourante prédéterminée de l'ensemble de filaments (3) pour anéantir le volume de tissus (9) enfermé par l'ensemble de filaments (3).
